(19) Europäisches Patentamt
European Patent Office
Office européen des brevets

(11) Veröffentlichungsnummer: **0 002 662**
**B1**

(12) # EUROPÄISCHE PATENTSCHRIFT

(45) Veröffentlichungstag der Patentschrift: **06.01.82**

(51) Int. Cl.³: **C 07 D 233/76,** C 07 D 233/86, C 08 G 73/06

(21) Anmeldenummer: **78101272.9**

(22) Anmeldetag: **02.11.78**

(54) **Verfahren zur Herstellung von (Thio)Hydantoinen.**

(30) Priorität: **29.12.77 DE 2758569**

(43) Veröffentlichungstag der Anmeldung: **11.07.79 Patentblatt 79/14**

(45) Bekanntmachung des Hinweises auf die Patenterteilung: **06.01.82 Patentblatt 82/1**

(84) Benannte Vertragsstaaten: **DE FR GB**

(56) Entgegenhaltungen:
**DE-A-1 670 970**
**DE-A-1 720 624**
**DE-A-1 906 492**
**DE-A-2 358 504**
**DE-A-2 539 730**
**FR-A-1 484 694**

(73) Patentinhaber: **BAYER AG, Zentralbereich Patente, Marken und Lizenzen, D-5090 Leverkusen 1, Bayerwerk (DE)**

(72) Erfinder: **Zecher, Wilfried, Dr., Treptower Strasse 6, D-5090 Leverkusen 1 (DE)**
Erfinder: **Lewalter, Jürgen, Dr., Bergstrasse 88, D-5068 Odenthal (DE)**
Erfinder: **Merten, Rudolf, Dr., Berta-von-Suttner-Strasse 55, D-5090 Leverkusen 1 (DE)**
Erfinder: **Dünwald, Willi, Dr., Geschwister-Scholl-Strasse 16, D-5090 Leverkusen 1 (DE)**

**EP 0 002 662 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von (Thio)Hydantoinen

Die Erfindung betrifft ein Verfahren zur Herstellung von Kondensaten mit mindestens einem Hydantoin- oder Thiohydatoinring im Molekül, aus ungesättigten Carbonsäuren und organischen Isocyanaten.

Verfahren zur Herstellung von Hydantoinen (J. Am. Chem. 45/383) bzw. Polyhydantoinen (BE 678 282) sind bekannt. Niedermolekulare Hydantoine werden bevorzugt im Pharma- und Pflanzenschutzbereich, höhermolekulare Hydantoine beispielsweise im wärmebeständigen Beschichtungsmittelsektor verwendet (franz. Pat. 1 484 694).

Überraschenderweise wurde gefunden, daß durch Umsetzung organischer Iso(thio)cyanate mit ungesättigten Carbonsäuren der allgemeinen Formel (I):

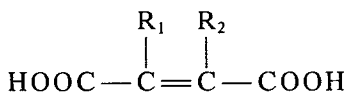

(I)

in der

$R_1$ und $R_2$ gleich oder verschieden, Wasserstoff, Halogen, einen gegebenenfalls substituierten aliphatischen, aliphatisch-aromatischen, aromatischen oder heterocyclischen Rest bedeuten, bei Temperaturen von $-20$ bis 500°C durch Amidgruppen modifizierte (thio)Hydantoine erhalten werden.

Der Umsatz von Carbonsäuren mit Isocyanaten liefert bekanntlich bevorzugt die entsprechenden Säureanhydride und Harnstoffe, die mit weiteren Isocyanaten zu komplizierten, z. T. auch polymeren Gemischen reagieren können. Überraschenderweise führt jedoch der Einsatz von mindestens 2 Val Isocyanat pro Mol der ungesättigten Carbonsäure zur Hydantoinringbildung.

Der glatte Ablauf der in mehreren Stufen verlaufenden Reaktion, der auch den Aufbau von Polymeren ermöglicht, war nicht zu erwarten, da jede Stufe für sich, speziell in Gegenwart überschüssiger Isocyanatmengen, zu einer Vielzahl komplizierter Stoffgemische reagieren kann. Schließlich werden im Zuge der erfindungsgemäßen Umsetzung nur $CO_2$, nicht jedoch auch Wasser oder Alkohol freigesetzt und somit Nebenreaktionen der bekannten Hydantoinherstellungsverfahren vermieden.

Die erfindungsgemäße Reaktion kann z. B. für Monoisocyanate durch die nachfolgende Gleichung wiedergegeben werden:

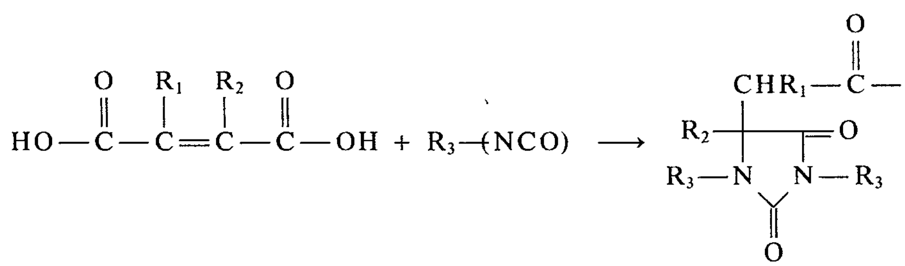

(I)

Die Reste $R_1$ und $R_2$ haben die oben angegebene Bedeutung und $R_3$ wird nachfolgend definiert. Bei der erfindungsgemäßen Reaktion können auch andere Reaktionsprodukte, z. B. Hydantoincarbonsäuren, in Abhängigkeit von den Reaktionsbedingungen neben den Amid-Gruppen modifizierten Hydantoinen entstehen.

Als Iso(thio)cyanate werden z. B. Verbindungen der allgemeinen Formel

$$R_3\text{—}(NCQ)_n$$

eingesetzt, in der $R_3$ einen gegebenenfalls substituierten n-wertigen aliphatischen, aliphatisch-aromatischen, aromatischen oder heterozyklischen Rest, n eine ganze Zahl von 1–4, vorzugsweise 1–2 und Q Sauerstoff oder Schwefel bedeuten.

Pro Mol der ungesättigten Dicarbonsäure können 2–12 Val, vorzugsweise 3–6 Val Iso(thio)cyanat eingesetzt werden, doch stört auch überschüssiges Iso(thio)cyanat den Verlauf der Reaktion nicht. Für $n = 1$ werden monomolekulare Hydantoine der Formel I, für $n > 1$ in Abhängigkeit von den stöchiometrischen Verhältnissen der Ausgangsmaterialien oligomere oder polymere Hydantoine mit Molgewichten bis 200 000 erhalten. Zum Aufbau der Oligomeren oder Polymeren werden mindestens 3 Val eines polyfunktionellen Iso(thio)cyanats eingesetzt.

Nach einer bevorzugten Ausführungsform werden pro Mol der ungesättigten Carbonsäure

mindestens 2, vorzugsweise 2,5–3 Mol, eines Diiso(thio)cyanats eingesetzt. Man erhält so Polyiso(thio)cyanate mit der gegebenenfalls 1–1000 mal, vorzugsweise 1–200 mal wiederkehrenden Struktureinheiten (II)

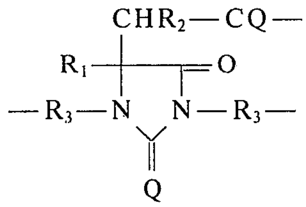

die gegebenenfalls über $R_3$-Reste verknüpft ist, und gegebenenfalls verkappten NCQ-Endgruppen. Die Reste $R_1$, $R_2$ und $R_3$, Q haben die oben angegebene Bedeutung. (Thio)Hydantoiniso(thio)cyanate aus dieser Reihe sind geeignet als Komponenten und Vernetzer z. B. in Polyestern, Polyurethanen, Polyhydantoinen und Polyimiden.

Die erfindungsgemäßen (Thio)Hydantoine können über die charakteristischen IR-Banden der (Thio)Hydantoine indentifiziert werden. Die höhermolekularen (Thio)Hydantoine besitzen in 30 Gew.-%igen Lösungen mit z. B. Acetophenon, Butylrolacton, Kresol oder Benzoesäurealkylester bei 20°C Lösungsviskositäten von 50 bis 200 000 mPa s, vorzugsweise von 500 bis 50 000 mPa s.

Im einzelnen sind die als Ausgangsmaterialien verwendeten ungesättigten Carbonsäuren Verbindungen der allgemeinen Formel (I)

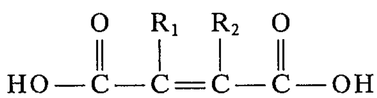

in der vorzugsweise

$R_1$ und $R_2$ gleich oder verschieden, Wasserstoff, Halogen, einen aliphatischen Rest mit $C_1-C_{20}$, aliphatisch-aromatischen Rest mit $C_7-C_{20}$, aromatischen Rest mit $C_6-C_{20}$ oder heterocyclischen Rest mit 5 bis 16 Ringgliedern und wenigstens einem N, O oder S-Atom bedeutet.

$R_1$ und $R_2$ können beispielsweise Wasserstoff, Fluor, Chlor, Brom, ein Methan-, Äthan-, Hexan-, Cyclohexan-, Propen-, Benzol-, Toluol-, Piperidin-, Morpholin- und Imidazolrest sein und gegebenenfalls zusammen einen Ring mit bis zu acht Gliedern bilden.

Die ungesättigten Dicarbonsäuren können auch durch in situ-Umsetzung von entsprechenden Anhydriden mit tertiärem Alkohol hergestellt werden. Es können also cis- und trans-Formen der ungesättigten Dicarbonsäuren verwendet werden, die ggf. in situ unter Verwendung bekannter Verfahren, z. B. durch Bestrahlung, Erhitzen, Halogen oder Basen erhalten werden. Besonders bevorzugt sind $R_1$ und $R_2$ Wasserstoff, d. h. die Isomeren Maleinsäure und Fumarsäure.

Als organische Iso(thio)cyanate können Mono- und/oder Polyiso(thio)cyanate verwendet werden.

Als Monoiso(thio)cyanate im Sinne der Erfindung werden aliphatische und aromatische, gegebenenfalls durch Heteroatome substituierte Verbindungen mit einer NCQ-Gruppe im Molekül eingesetzt, z. B. Alkylisocyanate wie Äthyl-, Methyl-, Butyl-, Dodecyl- und Stearylisocyanat, aromatische, gegebenenfalls substituierte Monoisocyanate wie Phenyl-, Tolyl-, Isopropyl-, Nonylphenylisocyanat, Nitro-, Alkoxy-, Aroxy-, Chlor-, Dichlor-, Trichlor-, Tetrachlor-, Pentachlor-, Benzyl-, Brom-phenyl-isocyanat oder Isocyanatobenzoesäureester, -phthalsäureester, -isophthalsäureester, Isocyanatobenzonitril, cycloaliphatische Isocyanate wie Cyclohexylisocyanat und ungesättigte Isocyanate wie Allyl-, Oleyl-, Cyclohexenylisocyanat.

Als erfindungsgemäß einzusetzende Ausgangskomponenten kommen ebenso aliphatische, cycloaliphatische, araliphatische, aromatische und heterocyclische Polyisocyanate in Betracht (vgl. Annalen 562, Seiten 75 bis 136).

Es ist auch möglich, die bei der technischen Isocyanatherstellung anfallenden Isocyanatgruppen aufweisenden Destillationsrückstände, gegebenenfalls gelöst in einem oder mehreren der vorgenannten Polyisocyanate, einzusetzen. Ferner ist es möglich, beliebige Mischungen der vorgenannten Polyisocyanate zu verwenden.

Vorzugsweise eignen sich Iso(thio)cyanate der allgemeinen Formel (III)

$$R_3(-NCQ)_n \qquad \text{(III)}$$

in den Q für O oder S und $R_3$ für einen, gegebenenfalls mit Halogen, Alkyl- mit $C_1-C_{10}$ und/oder Arylgruppen mit $C_6-C_{12}$ substituierten Alkylrest mit 2–20 C-Atomen, einen Arylrest mit 5–12 C-Atomen, einen Cycloalkylrest mit 5–12 C-Atomen, einen Alkyl-Arylrest mit 6–20 C-Atomen und

einen Heteroatome wie N, O oder S enthaltenden Aryl- oder Cycloalkylrest mit 5 – 12 C-Atomen steht. n ist eine ganze Zahl von 1 – 4, vorzugsweise 1 – 3, besonders bevorzugt 2. Besonders bevorzugt sind aliphatische Reste mit 2 – 12 C-Atomen oder ein Arylrest wie Phenyl, Tolyl, Naphthyl, Diphenylmethan- und Diphenylätherreste.

Bevorzugt verwendet werden die technisch leicht zugänglichen Gemische aus Toluylen-diisocyanaten, m-Phenylendiisocyanat, sowie phosgenierte Kondensate aus Anilin und Formaldehyd mit Polyphenylen-methylen-struktur und die symmetrischen Verbindungen 4,4'-Diisocyanatodiphenylmethan, 4,4'-Diisocyanatodiphenyläther, p-Phenylendiisocyanat und 4,4'-Diisocyanatodiphenyldimethylmethan sowie Isophorondiisocyanat und Hexamethylendiisocyanat.

Die Iso(thio)cyanate können in freier Form, ferner zum Teil oder vollständig auch in Form ihrer beim Umsatz mit reaktiven Wasserstoff enthaltenden Verbindungen zugänglichen und unter den Reaktionsbedingungen als Isocyanat-Abspalter reagierenden Derivate eingesetzt werden.

Vorzugsweise werden als Abspalter die Additionsprodukte aus Lactamen, Oximen und CH-aciden Verbindungen sowie die aus aliphatischen und aromatischen Mono- und Polyhydroxy-Verbindungen erhaltenen Carbamidsäureester, beispielsweise der allgemeinen Formeln

$$R_3\left(-NH-\underset{\underset{O}{\|}}{C}-O-M\right)_n$$

bzw.

$$\left[-\underset{\underset{O}{\|}}{C}-NH-R_3-NH-\underset{\underset{O}{\|}}{C}-O-L-O-\right]_y$$

eingesetzt, worin $R_3$ und n die oben angegebene Bedeutung haben und M der organische Rest einer Monohydroxyverbindung bzw. L der organische Rest einer bis- oder trisfunktionellen Hydroxyverbindung, vorzugsweise M und L, gleich oder verschieden, ein aliphatischer Rest mit 1 – 10 C-Atomen, ein cycloaliphatischer Rest mit 5 – 10 C-Atomen, ein aliphatisch-aromatischer Rest mit 7 – 12 C-Atomen und ein aromatischer Rest mit 6 – 12 C-Atomen ist, die jeweils noch mit Alkyl- mit $C_1 - C_{10}$ und/oder Arylgruppen mit $C_6 - C_{12}$ substituiert sein können, und y für eine ganze Zahl von 1 bis 1000, vorzugsweise von 1 bis 100 steht.

Als Beispiele seien die Carbamidsäureester aus Phenol, isomeren Kresolen, deren technischen Gemischen und ähnlichen aromatischen Hydroxylverbindungen, aliphatische Monoalkohole wie Methanol, Äthanol, Propanol, Isopropanol, Butanol, Isobutanol, Cyclohexanol, Allylalkohol, Benzylalkohol und aliphatische Di- oder Polyole wie Äthylenglykol und Trimethylolpropan, weiterhin die Additionsprodukte mit Pyrrolidon-(2), Caprolactam, Butanon-oxim, Malonester, Acetessigester und Acetophenon aufgeführt.

Die Isocyanat-Abspalter können als solche eingesetzt oder erst in situ durch Umsetzung mit den entsprechenden Reaktanten erzeugt werden.

Anstelle der genannten (Poly)Isocyanate können auch die analogen (Poly)-Isothiocyanate als Ausgangsmaterialien benutzt werden.

Die erfindungsgemäß als Blockierungs- wie auch Lösungsmittel besonders bevorzugten Hydroxylalkyläther sind beispielhaft Verbindungen der allgemeinen Formel (IV)

$$R_8-(OR_9)_q-OH \qquad (IV)$$

in der $R^8$ einen gegebenenfalls substituierten aliphatischen Rest mit $C_1 - C_{20}$, vorzugsweise $C_1 - C_8$, cycloaliphatischen Rest mit $C_4 - C_{10}$, vorzugsweise $C_5 - C_8$, aliphatisch-aromatischen Rest mit $C_7 - C_{16}$ oder aromatischen Rest mit $C_6 - C_{14}$, der z. B. mit Alkoxy-, Aroxy- oder Hydroxy-Gruppen substituiert sein kann, $R^9$ einen aliphatischen Rest mit 2 – 20 C-Atomen und q eine ganze Zahl von 1 – 100, bevorzugt 1 – 4, bedeuten. Vorzugsweise werden erfindungsgemäß solche Hydroxylalkyl-Äther eingesetzt, die pro Molekül eine Hydroxy-Gruppe enthalten und in denen $R^9$ einen Rest mit zwei C-Atomen in der Kette, die beispielsweise durch Alkyl-Gruppen substituiert sein können, bedeutet, z. B. die Methyl-, Isopropyl-, Cyclohexyl-, Benzyl-, Phenyl- und Methoxy-äthyl-äthylenglykol- und -propylenglykol- bzw. diäthylenglykol und -dipropylenglykol-monoäther.

Der erfindungsgemäße Umsatz der ungesättigten Carbonsäuren mit den organischen Iso(thio)cyanaten zu den beanspruchten (Thio)Hydantoinen bzw. (Thio)Hydantoingruppen enthaltenden Polyiso(thio)cyanaten kann in Lösungsmitteln, die unter den Reaktionsbedingungen nicht reagieren oder lockere, weiterreagierende Additionsverbindungen bilden, oder in einem Überschuß einer der Reaktionskomponente durchgeführt werden.

Außer den bereits aufgeführten Blockierungsmitteln eignen sich als Lösungsmittel Kohlenwasserstoffe, Halogenkohlenwasserstoffe, Ester, cyclische Ester, Ketone, Äther, substituierte Amide, Nitrile, beispielsweise Xylole, o-Dichlorbenzol, Acetophenon, Cyclohexanon, Äthylenglykolbutyläther, Diäthylenglykolmethyläther, Glykolmonomethylätheracetat, $\gamma$-Butyrolacton, $\varepsilon$-Caprolacton, Benzoesäurealkylester, N-Methylpyrrolidon, Dimethylformamid, Dimethylacetamid, Benzonitril und andere sowie deren Gemische.

Zur Durchführung der erfindungsgemäßen Verfahren werden die Reaktionskomponenten, mit oder ohne Lösungsmittel und/oder Blockierungsmittel, einige Minuten bis zu mehreren Stunden bei Temperaturen von −20°C bis 500°C, bevorzugt 0°C bis 450°C, gehalten. Der Reaktionsverlauf kann über die Gasentwicklung und die IR-Spektren verfolgt werden.

Die Acidität saurer Lösungsmittel wie der Phenole bzw. Kresole genügt zur Reaktionsführung innerhalb hinreichend kurzer Reaktionszeiten. In inerten Medien oder in Schmelzansätzen können beispielsweise Carbonsäuren mit hinreichendem Schmelz- bzw. Siedepunkt wie Essigsäure, Benzoesäure, Bernsteinsäure, Benzoldicarbonsäuren, Butantetracarbonsäure, Trimelitsäure bzw. deren Anhydride gegebenenfalls auch als einbaufähige Katalysatoren in Mengen von 0,1 bis 40, vorzugsweise von 1 bis 10 Prozent in Val, bezogen auf ein Val Isocyanat, mitverwendet werden.

Zur Beschleunigung der ablaufenden Reaktionen sind die aus der Isocyanatchemie bekannten Katalysatoren wie Basen, beispielsweise Triäthylamin, N-Methylmorpholin, Endoäthylenpiperazin, wie Säuren, beispielsweise p-Toluolsulfonsäure wie Metalle, insbesondere von Eisen, Blei, Zink, Zinn, Kupfer, Kobalt, Titan, beispielsweise Titantetrabutylat, Titanaminoalkohol, Eisenacetylacetonat, Dibutylzinnlaurat, Bleiacetat, Zinkoctoat oder Phosphorverbindungen wie Trialkylphosphin, zu verwenden.

Zuweilen ist es vorteilhaft, die Reaktion in mehreren Stufen durchzuführen. Durch stufenweise Temperaturführung und durch stufenweise Zugabe, gegebenenfalls verschiedener Isocyanate kann ein definierter Aufbau der erfindungsgemäßen Verfahrensprodukte erreicht werden.

So kann beispielsweise in einer ersten Stufe, gegebenenfalls in einem Lösungs- und/oder Blockmittel ein Addukt oder Kondensat hergestellt werden, das dann bei höherer Temperatur, eventuell unter Verdampfung des Lösungsmittels, gegebenenfalls nach Zusatz latenter Blockierungsmittel unter Cyclisierung und/oder Kettenverlängerung und/oder Vernetzung in das gegebenenfalls hochmolekulare Kondensationsprodukt übergeht. Wird dieses dann zur Beschichtung verwendet, so kann es auch aus Schmelzen oder wäßrigen Systemen appliziert werden.

Mitunter wird die Umsetzung zweckmäßigerweise unter einem inerten Schutzgas wie $N_2$ oder Argon durchgeführt.

Schließlich kann die erfindungsgemäße Reaktion in kontinuierlicher oder diskontinuierlicher Ausführung oder gegebenenfalls in Autoklaven unter Druck zum Zwecke einer höheren Reaktionstemperatur erfolgen.

Im allgemeinen wird entsprechend der erfindungsgemäßen Reaktion, vorteilhafterweise pro Mol ungesättigter Dicarbonsäure mindestens 2 Val, vorzugsweise 3−6 Val, Iso(thio)cyanat eingesetzt.

Durch Mitverwendung von Monoisocyanaten wie z. B. Phenylisocyanat, $\alpha$-Naphthylisocyanat, Isocyanatobenzoesäureester, Isocyanatoessigsäureester können der Polymerisationsgrad und Isocyanatgehalt variiert werden.

Die Aufarbeitung der Reaktionsprodukte kann nach gängigen Methoden wie z. B. Kristallisation erfolgen.

Eine weitere Ausführung der erfindungsgemäßen Reaktion besteht darin, daß ggf. in situ beispielsweise mehrwertige Amine, mehrwertige Alkohole wie beispielsweise Äthylenglykol, Trimethylolpropan, Glycerin, Trishydroxyäthylisocyanurat und/oder mehrwertige Carbonsäuren bzw. deren Anhydride wie beispielsweise Phthalsäure, Isophthalsäure, Terephthalsäure, Trimellitsäure, Butantetracarbonsäure und/oder weitere Polyisocyanate bzw. Polyisocyanat-Abspalter, mitverwendet und in der üblichen Weise zu linearen und/oder verzweigten Kunststoffen mit z. B. Ester, Carbamidester, Amid- und/oder Imid-Gruppen von guter Löslichkeit, höherer Temperaturbeständigkeit, guter Elastizität und gutem Hitzeschockverhalten umgesetzt werden können.

Mit analogem Erfolg können die erfindungsgemäßen Kondensationsprodukte, gegebenenfalls auch ihre Vorstufen, Polyestern z. B. aus Phthalsäure/Terephthalsäure/Isophthalsäure, bzw. deren Ester, Äthylenglykol, Glycerin/Trimethylolpropan/Trishydroxyäthylisocyanurat, Polyäthern z. B. aus Äthylenoxid und/oder Bis(hydroxyphenyl)-propan und Epichlorhydrin, Polyurethanen, Polyamiden, Polyimiden, Polyester usw. zugemischt und gegebenenfalls einkondensiert oder die Reaktion in Gegenwart dieser Komponenten durchgeführt werden. In allen Fällen entstehen dabei modifizierte Polymere, die neben den (Thio)-Hydantoinringen gegebenenfalls zusätzliche Äther-, Carbamidester-, Carbonsäureester, -amid-, -imid-, -esteramid-, -esterimid-, -amidimid- und/oder -esteramidimidgruppierungen enthalten.

Die Mengenverhältnisse dieser Zusätze können in weiten Bereichen schwanken, vorzugsweise werden bezüglich des erfindungsgemäßen Kondensats 10−400 Gew.-% eingesetzt.

Die nach den erfindungsgemäßen Verfahren zugänglichen nieder- bzw. monomolekularen Hydantoine besitzen biochemische Wirkungen, die erfindungsgemäßen Polyhydantoine eine besondere Temperaturbeständigkeit.

Die mit der erfindungsgemäßen Polykondensation modifizierten Kunststoffe zeigen verbesserte

Temperaturverhalten sowie verbesserte Löslichkeit. Die Polymeren können zur Herstellung temperaturbeständiger Kleber, Lacke, Pulver, Folien und Kunststoffe sowie zur Beschichtung hitzeresistenter Substrate verwendet werden. Ihre Eigenschaften können je nach Einsatzgebiet durch Zusatz von Füllstoffen, Pigmenten, nieder- und hochmolekularen Komponenten in weiten Grenzen variiert werden.

Beispiel 1

In 570 g Butyrolacton werden 58 g Fumarsäure und 375 g 4,4'-Diisocyanato-diphenylmethan eingetragen. Dann wird auf 110°C aufgeheizt und bei dieser Temperatur nach Maßgabe der exothermen Reaktion 111 g n-Butanol zugetropft. Anschließend wird 3 Stdn. bei 130°C, 3 Stdn. bei 150°C und 7 Stdn. bei 170°C gerührt. Die Kondensation zum Hydantoin verläuft unter Abspaltung von Kohlendioxid. Man erhält eine braune viskose Lösung des mit Butanol blockierten Hydantoin-isocyanats, deren Isocyanatgehalt <0,5% ist und die im IR-Spektrum bei 1720 und 1770 $cm^{-1}$ die für Hydantoine charakteristischen Banden zeigt.

Zur Bereitung einer Lacklösung werden 200 g des Reaktionsproduktes mit 100 g eines Polyesters aus Terephthalsäure, Äthylenglykol und Glycerin abgemischt, mit Kresol auf einen Festgehalt von 30% verdünnt und mit 0,5% Titantetrabutylat als Katalysator versetzt. Auf einer Drahtlackiermaschine wird mit dieser Lösung ein Cu-Draht von 0,7 mm Ø lackiert.

| | |
|---|---|
| Ofenlänge: | 4 m |
| Ofentemperatur: | 400°C |
| Anzahl der Durchzüge: | 6 |

Unter diesen Bedingungen wird bei einer Abzugsgeschwindigkeit des Drahtes von 9 m/Min. ein beschichteter Draht mit einer Erweichungstemperatur von 324°C und einer Abriebfestigkeit von 20 Hüben erhalten.

Beispiel 2

In eine Lösung von 375 g 4,4'-Diisocyanato-diphenylmethan und 58 g Fumarsäure in 570 g Butyrolacton werden bei 120°C 168 g eines technischen Kresolgemisches eingetropft. Dann wird jeweils 2 Stdn. bei 130°C, 150°C, 170°C und 175°C gerührt. Das mit Kresol verkappte Hydantoin-isocyanat wird als braune Lösung mit den für Hydantoin typischen Banden im IR-Spektrum bei 1720 und 1775 $cm^{-1}$ erhalten. Der Gehalt an verkapptem Isocyanat beträgt 5,6%, die Viskosität $\eta^{25}$ 360 m Pas.

Das Reaktionsprodukt wird im Verhältnis 1 : 1 mit einem Polyester aus Terephthalsäure, Äthylenglykol und Glycerin abgemischt, mit Kresol auf einen Festgehalt von 30% verdünnt und mit 1,5% Titantetrabutylat als Katalysator versetzt. Mit dieser Lösung wird, wie in Beispiel 1 beschrieben, ein Cu-Draht von 0,7 mm Ø lackiert. Unter diesen Bedingungen wird bei einer Abzugsgeschwindigkeit von 11 m/Min. ein isolierter Draht mit einer max. Außenfaserdehnung von 88% und einer Erweichungstemperatur von 318°C erhalten.

Beispiel 3

58 g Fumarsäure werden in 600 g eines technischen Kresolgemisches vorgelegt und bei 120°C anteilweise mit 261 g eines Gemisches aus 80 Tln. 2,4- und 20 Tln. 2,6-Toluylendiisocyanat versetzt. Dann wird 2 Stdn. bei 150°C, 2 Stdn. bei 170°C und 6 Stdn. bei 190°C gerührt. Die Kondensation zum Hydantoin erfolgt unter Abspaltung von Kohlendioxid. Das mit Kresol verkappte Hydantoin-isocyanat wird als braune viskose Lösung mit einem Gehalt an verkapptem Isocyanat von 6,7% erhalten. Eine Probe wird im Gewichtsverhältnis 1 : 1 mit einem Polyester aus Isophthalsäure, Äthylenglykol und Trimethylolpropan abgemischt und mit Acetophenon auf einen Festgehalt von ca. 30% verdünnt. Diese Lacklösung wird auf ein Blech aufgestrichen und in jeweils 15 Min. bei 200 und 300°C zu einem klaren elastischen Lackfilm eingebrannt.

Beispiel 4

96 g Solvesso 100 (technisches Alkylaromaten-Gemisch), 58 g Fumarsäure und 261 g eines Gemisches aus 80 Tln. 2,4- und 20 Tln. 2,6-Toluylendiisocyanat werden vorgelegt und auf 100°C erhitzt. Dann werden bei dieser Temperatur 180 g Diäthylenglykolmonomethyläther unter Kühlung zugetropft. Anschließend wird jeweils 2 Stdn. bei 120, 150 und 170°C und 4 Stdn. bei 190°C gerührt. Das restliche Solvesso, das im Verlaufe der Reaktion noch nicht übergegangen ist, wird unter Vakuum abdestilliert.

Das Reaktionsprodukt, ein mit Diäthylenglykolmonomethyläther verkapptes Hydantoin-isocyanat, wird als Schmelze ausgegossen und erstarrt zu einem gelbbraunen Harz. Der Gehalt an freiem Isocyanat beträgt <0,2%. Aus 200 g dieses Harzes und 200 g eines Polyesters aus Terephthalsäure, Äthylenglykol und Glycerin wird eine 30%ige Lösung in Kresol bereitet, die mit 1,5% Titantetrabutylat als Katalysator versetzt wird. Mit dieser Lacklösung wird unter den in Beispiel 1 beschriebenen Bedingungen ein Cu-Draht von 0,7 mm Ø lackiert. Es wird bei einer Abzugsgeschwindigkeit von 9 m/Min. ein Lackdraht mit einer Erweichungstemperatur von 310° C und einer Abriebfestigkeit von 19 Hüben erhalten.

## Beispiel 5

In die Lösung von 152 g eines mit Kresol verkappten Hydantoin-isocyanats, das nach Beispiel 2 hergestellt wurde, und 31 g Äthylenglykol in 255 g eines technischen Kresolgemisches werden bei 110–120° C 70 g 2,4-Toluylendiisocyanat eingetropft. Dann wird 30 Min. bei 120° C nachgerührt und 192 g Trimellitsäureanhydrid eingetragen. Anschließend wird jeweils 2 Stdn. bei 170, 190, 200 und 205° C gerührt. Die Kondensation erfolgt unter Abspaltung von Kohlendioxid und Wasser. Das Reaktionsprodukt, ein Polyesterimid-hydantoin mit charakteristischen Banden im IR-Spektrum bei 1715 und 1775 $cm^{-1}$, wird mit 150 g einer Mischung aus gleichen Teilen Phenol und Kresol verdünnt. Die Viskosität $\eta^{25}$ beträgt 13 000 m Pas. Eine Probe wird mit Kresol auf 30 Gew.-% verdünnt und mit gleichen Anteilen, bezogen auf den Festgehalt, eines Polyesters aus Terephthalsäure, Äthylenglykol und Glycerin und 1,5% Titantetrabutylat als Katalysator versetzt. Diese Lacklösung wird auf eine Glasplatte aufgestrichen und in jeweils 15 Min. bei 200 und 300° C zu einem klaren elastischen Lackfilm eingebrannt.

## Beispiel 6

In 58 g Fumarsäure in 600 g Kresol werden bei 120° C 261 g eines Gemisches aus 80 Tln. 2,4- und 20 Tln. 2,6-Toluylendiisocyanat unter Kühlung eingetropft. Dann wird 8 Stdn. bei 190° C gerührt. Anschließend wird auf 120° C abgekühlt und bei dieser Temperatur 85 g Äthylenglykol, 130 g 2,4-Toluylendiisocyanat und 524 g Trimellitsäureanhydrid eingetragen. Danach wird aufgeheizt und jeweils 2 Stdn. bei 190, 200 und 210° C gerührt. Das Reaktionsprodukt, ein Poly-ester-imid-hydantoin, wird mit 650 g Kresol zu einer ca. 40%igen Lösung verdünnt. Die Viskosität $\eta^{25}$ einer 15%igen Lösung in Kresol beträgt 400 m Pas. Eine Probe des Reaktionsproduktes wird auf ein Blech aufgestrichen und in jeweils 15 Min. bei 200 und 300° C zu einem klaren harten Lackfilm eingebrannt.

## Beispiel 7

| | |
|---|---|
| 1000,8 g | 4,4'-Diisocyanatodiphenylmethan unter $N_2$ ab 50–150° C portionsweise mit der zuvor aus |
| 232,2 g | Maleinsäure |
| 562,8 g | Carbitol (Diäthylenglykolmonoäthyläther) |
| 18 g | ε-Caprolactam unter $N_2$ bei 30–70° C durch Eintragen von |
| 222 g | Isophorondiisocyanat hergestellten ($CO_2\uparrow$), NCO-freien Lösung versetzen, |
| | 1 Stunde bei 150° C homogenisieren und schließlich ca. 4 Stunden bei 200–220° C (Rückfluß) kondensieren. |
| | Das schwarz-braune, homogene Harz besitzt die Hydantointypischen IR-Banden, hat einen latenten NCO-Gehalt von ca. 9,0% und eine Viskosität von ca. 20 000 $cP_{20°C}$ in 70%iger Carbitollösung. |

## Beispiel 8

| | |
|---|---|
| 522 g | Toluylendiisocyanat (Isomerengemisch 2,4 : 2,6 wie 80 : 20) werden unter $N_2$ bei 30–70° C mit der Mischung aus |
| 116,1 g | Fumarsäure |
| 10 g | ε-Caprolactam |
| 340 g | Benzylalkohol versetzt, |
| | dann über 100, 120, 150 auf 160–175° C erhitzt und nach dem Abklingen der $CO_2$-Entwicklung noch 1 Stunde bei 200° C kondensiert. |
| | Das schwarz-braune, spröde Harz besitzt die Hydantointypischen IR-Banden, hat eine Viskosität von ca. 250 $cP_{150°C}$ und enthält einen latenten NCO-Gehalt von ca. 13 Gew.-%. |

Beispiel 9

75,0 g 4,4'-Diisocyanatodiphenylmethan werden unter $N_2$ bei 30 bis 70°C mit der zuvor kurz auf 140 – 150°C angeheizten Mischung aus
11,6 g Maleinsäure
1 g ε-Caprolactam
42 g Carbitol
0,05 g Endoäthylenpiperazin
0,05 g Jod
vermischt, homogenisiert,
dann über 100/120/150 auf 160 – 175°C und nach dem Abklingen der $CO_2$-Entwicklung kurz auf 190 – 210°C erhitzt.
Anschließend verdünnt man die NCO-freie Schmelze bei 120 – 100°C mit
970 g Xylenol L(R), versetzt mit
520 g eines Polyesters aus 5,7 Mol Dimethylterephthalat, 1,9 Mol Tris-(2-hydroxyäthyl)-isocyanurat, 0,6 Mol Trimethylolpropan, 10,0 Mol Äthylenglykol, 300 g Solvesso, 1,0 g Bleiacetat und 1,0 g Butyltitanat mit einem Hydroxylgruppen-Gehalt von ca. 4,5 Gew.-%, homogenisiert in ca. 1/2 Stunde bei 180 – 200°C, und gibt dann bei 100 – 80°C
6,5 g Titantetrabutylat hinzu.
Die Viskosität der ca. 40%igen Lacklösung liegt bei ca. 1700 $cP_{20°C}$.
Die mit ihr in einem 4 m Ofen bei 9 m/Min. beschichteten Cu-Drähte von 0,7 mm besitzen eine Erweichungstemperatur von ca. 315 – 320°C, einen Hitzeschock von mindestens 220°C und eine Wärmebeständigkeit bei 200°C von ca. 14 Tagen.

Beispiel 10

19,3 g Maleinsäure
0,2 g Jod in
500 g m Kresol 70 werden unter $N_2$ ca. 1 Minute auf 140 – 150°C erhitzt, dann bei 50°C mit
298,9 g N,N'-Bis-(2-methoxycarbonylpropyl-2)-4,4-diamino-diphenylmethan versetzt, und schließlich ab 30 – 45°C rasch mit der Lösung von
250,2 g 4,4'-Diisocyanatodiphenylmethan in
200 g Toluol vermischt. Nach ca. 3 Stunden bei 25°C – 35°C versetzt man mit
0,5 g Dabco (Endoäthylenpiperazin), heizt über 100/120/140/160 auf 175°C, trägt dabei $CO_2$, Methanol/Toluol aus und kondensiert schließlich ca. 6 Stunden bei 200°C.
Im Zuge steigender Viskosität wird mit insgesamt
680 g m Kresol 70 verdünnt und 1 Stunde bei 200°C homogenisiert.
Die ca. 30%ige Lacklösung besitzt eine Viskosität von 4750 cP 20°C.
Das nach einer Methanolfällung verbleibende Bindemittel besitzt im IR-Spektrum die den Hydantoinen eigenen Banden.
Der in einem 4 m Ofen bei 9 m/Min. hergestellte 0,7 mm Cu-Lackdraht hat eine Erweichungstemperatur von >380°C, einen Hitzeschock von >260°C und eine Dauerwärmebeständigkeit von mindestens 14 Tagen 200°C.

Beispiel 11

77,8 g Maleinsäure
16,6 g Isophthalsäure
0,3 g Jod in
500 g γ-Butyrolacton werden unter $N_2$ ca. 1 Minute auf 140 – 150°C erhitzt, dann bei 30°C mit
298,9 g N,N'-Bis-(2-methoxycarbonylpropyl-2)-4,4'-diamino-diphenylmethan versetzt und schließlich bei 20°C in einem Schub mit der Lösung aus
663,0 g 4,4'-Diisocyanatodiphenylmethan in
200 g Toluol verrührt.
Nach ca. 3 Stunden bei 25°C – 35°C versetzt man mit
0,5 g Dabco, heizt langsam über 60/80/120/150 auf 175°C unter Austragung von $CO_2$, Methanol und Toluol.
Nach dem Abklingen der $CO_2$-Entwicklung werden bei 70°C
230,5 g Trimellitsäureanhydrid und
66,5 g Isophthalsäure hinzugefügt, homogenisiert und das Gemisch ca. 5 Stunden bei 200°C kondensiert.
Anschließend versetzt man bei 120°C mit
97,7 g Terephthalsäuredimethylester und

184,2 g Glycerin sowie
1,0 g Butyltitanat, heizt über 140/150° C ca. 6 Stunden auf 200 – 220° C und kondensiert zuletzt ca. 1 Stunde unter Vakuum bei 200 – 210° C.
Abschließend verdünnt man den Ansatz bei 150 – 120° C mit
955 g ε-Caprolacton und versetzt mit der Lösung von
14 g Titantetrabutylat in
28 g Acetylaceton und homogenisiert noch ca. 1 Stunde bei 120 – 100° C.
Die ca. 50%ige Lacklösung besitzt eine Viskosität von 17 300 $cP_{20°C}$.
Der in einem 4 m Ofen bei 9 m/Min. hergestellte 0,7 mm Cu-Lackdraht besitzt eine Erweichungstemperatur von >330° C (DIN 46 455), einen Hitzeschock von >260° C, eine Dauerwärmebeständigkeit von >7 Tagen bei 200° C (DIN 46 453), eine Schabefestigkeit (DIN 46 453) von 95 Doppelhüben, eine Lackfilmhärte von 5 H (DIN 46 453), eine Durchschlagfestigkeit von 9 KV, sowie eine gute Chemikalienbeständigkeit.

## Beispiel 12

76,8 g Trimellitsäureanhydrid und
8,3 g Isophthalsäure werden in
500 g m Kresol 70(R) unter $N_2$ in der Wärme gelöst, dann bei Raumtemperatur
298,9 g N,N'-Bis-(2-methoxycarbonylpropyl-2)-4,4'-diaminodiphenylmethan zugegeben und ab 30 – 45° C mit der Lösung von
725,6 g 4,4'-Diisocyanatodiphenylmethan in
200 g Toluol vermischt. Nach 3 Stunden bei Raumtemperatur gibt man noch
307,4 g Trimellitsäureanhydrid und
69,6 g Fumarsäure hinzu, homogenisiert, versetzt mit
0,5 g Dabco, heizt langsam über 70/100/120 auf 150° C und kondensiert nach dem Abklingen der $CO_2$-Entwicklung ca. 5 Stunden bei 200° C.
Abschließend versetzt man bei 120 – 100° C mit
97,7 g Terephthalsäuredimethylester
186 g Glykol und
1,0 g Butyltitanat und heizt über 140/150 auf 200° C.
Nach ca. 4 Stunden bei 200 – 220° C wird im Vakuum bei 200 – 210° C so weit kondensiert, daß eine mit Benzylalkohol auf 50%igen Festgehalt verdünnte Lacklösung eine Viskosität von ca. 2700 $cP_{20°C}$ besitzt.
Ein mit dieser Lacklösung beschichtetes und bei 250° C während 15 Min. und bei 300° C während 10 Min. eingebranntes Tiefziehblech erhält einen Lackfilm von ausgezeichneter Elastizität und Haftfestigkeit. Die Überzüge besitzen eine Bleistifthärte von 5 H. Andererseits kann dieser Lack z. B. auf Glas in an sich bekannter Weise zu einem Film verarbeitet werden. Die so hergestellten Folien besitzen gute mechanische Eigenschaften sowie eine hohe Wärmebeständigkeit mit einem Schmelzpunkt oberhalb 330° C.

## Beispiel 13

116,1 g Fumarsäure
16,6 g Isophthalsäure werden in
500 g γ-Butyrolacton unter $N_2$ ca. 1 Minute auf 100° C geheizt, dann bei 30° C mit der Lösung von
775,6 g 4,4'-Diisocyanatodiphenylmethan in
200 g Toluol vermischt und ca. 1 Stunde nachgerührt.
Nach Zusatz von
0,5 g Dabco heizt man langsam über 70/100 auf ca. 150° C, kühlt nach dem Abklingen der $CO_2$-Entwicklung auf ca. 70° C und versetzt mit
574,4 g Trimellitsäureanhydrid, heizt erneut über 70/100/150 auf ca. 175° C und kondensiert ca. 5 Stunden bei 200° C.
Anschließend wird bei 120 – 100° C mit
124,2 g Glykol
92,1 g Glycerin und
1,0 g Butyltitanat versetzt und über 140/150 auf 200° C geheizt. Nach ca. 4 Stunden bei 200 – 220° C wird im Vakuum bei 200 – 210° C bis auf eine Viskosität von ca. 67 000 $cP_{20°C}$ in einer mit Benzylalkohol auf 50% verdünnten Lacklösung kondensiert.
Ein mit dieser Lacklösung analog Beispiel 12 beschichtetes Tiefziehblech erhält einen elastischen und haftfesten Lackfilm von hoher Oberflächenhärte.

Beispiel 14

| | |
|---|---|
| 35,7 g | Phenylisocyanat werden mit |
| 70,0 g | m-Kresol vermischt und unter $N_2$ ab 10° C bis <40° C im Zuge der $CO_2$-Entwicklung mit |
| 11,6 g | Maleinsäure versetzt. Man gibt |
| 0,2 g | Jod hinzu, rührt ca. 1 Stunde bei 70° C nach, versetzt mit |
| 0,1 g | Dabco, heizt langsam über 100/130/150° C auf 170° C und steigert die Temperatur schließlich bis zum Verbrauch aller NCO-Gruppen bis zum Rückflußkochen. |

Zur Aufarbeitung wird das Kresol i. V. abdestilliert und der Rückstand aus Alkohol/Petroläther umkristallisiert. Es resultieren ca. 5 g farbloser Kristalle vom FP: 203° C – 205° C und den Hydantointypischen JR-Absorptionen bei 1710 und 1755 $cm^{-1}$ sowie einer Amidbande bei 1650 $cm^{-1}$.

Die auf 1,3-Diphenyl-5-(N-phenyl-aminocarbonylmethyl)-hydantoin berechnete Analyse fordert für $C_{23}H_{16}N_3O_3$ (385,4)

| | | | |
|---|---|---|---|
| ber.: | C 71,7, | H 5,0, | N 10,9, |
| gef.: | C 72,0, | H 5,1, | N 10,4. |

Beispiel 15

| | |
|---|---|
| 1392 g | Poluylendiisocyanat (Isomerengemisch 2,4 : 2,6 wie 80 : 20) werden unter $N_2$ bei Raumtemperatur mit |
| 116,1 g | Fumarsäure vermischt, mit |
| 0,1 g | Triäthylendiamin versetzt und nach Maßgabe der $CO_2$-Entwicklung langsam auf 175° C angeheizt. Man rührt ca. 3 Stunden bei 175° C bis zum Ende der $CO_2$-Entwicklung. |

Es resultiert ein dunkelbraunes Öl, dessen IR-Spektrum neben den Isocyanatbanden die den Hydantoinen eigenen Banden aufweist. Der Isocyanatgehalt des Öls beträgt ca. 31,0 Gew.-%.

Beispiel 16

| | |
|---|---|
| 24,5 g | Maleinsäureanhydrid und |
| 18,5 g | tert.-Butanol werden unter $N_2$ bis zum Ende der Isobutylenentwicklung (ca. 1 Stunde) gerührt, die resultierende Salbe dann in |
| 150 g | $\gamma$-Butyrolacton gelöst, homogenisiert, schließlich mit |
| 0,1 g | Jod verrührt und ca. 1 Stunde bei 70° C nachgerührt. Darauf versetzt man bei 10 – 15° C in einem Schub mit |
| 89,3 g | Phenylisocyanat, fügt |
| 0,4 g | Dabco zu und behandelt das Reaktionsgemisch in der unter Beispiel 14 beschriebenen Weise. |

Nach der Umkristallisation resultieren ca. 7 g eines farblosen Kristallisats vom FP: 205° C und der hydantointypischen IR-Absorption. Die auf 1,3-Diphenyl-5-(N-phenyl-aminocarbonylmethyl)-hydantoin berechnete Analyse fordert:

| | | | |
|---|---|---|---|
| ber.: | C 71,7, | H 5,0, | N 10,9, |
| gef.: | C 71,5, | H –, | N 10,6. |

**Patentansprüche**

1. Verfahren zur Herstellung von (Thio)Hydantoinen, bestehend aus der gegebenenfalls 1 – 1000mal wiederkehrenden Struktureinheit II

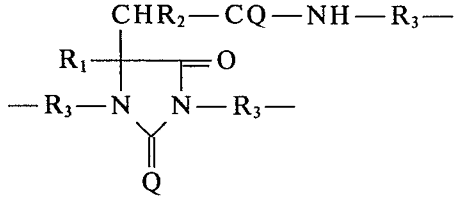

(II)

die gegebenenfalls über wenigstens einen $R_3$-Rest verknüpft ist, und gegebenenfalls verkappten NCQ-Endgruppen enthält, wobei die Reste

$R_1$, $R_2$ und $R_3$, gleich oder verschieden, einen gegebenenfalls substituierten aliphatischen, aliphatisch-aromatischen, aromatischen oder heterocyclischen Rest
$R_1$ und $R_2$ außerdem noch Wasserstoff und Halogen und
Q Sauerstoff oder S bedeuten,

dadurch gekennzeichnet, daß organische Iso(thio)cyanate der allgemeinen Formel (III)

$$R_3(-NCQ)_n$$

bzw. Iso(thio)cyanat-Abspalter mit ungesättigten Carbonsäuren der allgemeinen Formel I

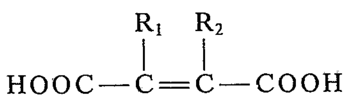

(I)

worin

$R_1$, $R_2$, $R_3$ und Q die oben angegebene Bedeutung hat und
n für eine ganze Zahl von 1 bis 4 steht,

gegebenenfalls in Anwesenheit eines Katalysators und einem oder mehreren Lösungsmitteln bei Temperaturen von $-20$ bis 500°C umgesetzt werden, wobei pro Mol ungesättigter Carbonsäure mindestens 2 Äquivalente Iso(thio)cyanat eingesetzt werden.

2. Verfahren nach Anspruch 1, dadurch gekennzeichnet, daß

$R_1$ und $R_2$, gleich oder verschieden, Wasserstoff, Halogen, einen aliphatischen Rest mit $C_1-C_{20}$, aliphatisch-aromatischen Rest mit $C_7-C_{20}$, aromatischen Rest mit $C_6-C_{20}$ oder heterocyclischen Rest mit 5 bis 16 Ringgliedern mit wenigstens einem N, O oder S-Atom im Ring,
$R_3$ einen Alkylrest mit 2–20 C-Atomen, einen Arylrest mit 5–12 C-Atomen, einen Cycloalkylrest mit 5–12 C-Atomen, einen Alkyl-Arylrest mit 6–20 C-Atomen und einen Heteroatome wie N, O oder S enthaltenden Aryl- oder Cycloalkylrest mit 5–12 C-Atomen, die alle mit Halogen, Alkyl mit $C_1-C_{10}$ und/oder Arylgruppen mit $C_6-C_{12}$ substituiert sein können, bedeuten.

3. Verfahren nach Anspruch 1, dadurch gekennzeichnet, daß organische Polyiso(thio)cyanate verwendet werden.

4. Verfahren nach Ansprüchen 1–3, dadurch gekennzeichnet, daß organische Diiso(thio)cyanate verwendet werden.

5. Verfahren nach Ansprüchen 1–4, dadurch gekennzeichnet, daß 3–6 Äquivalente Iso(thio)cyanat pro Mol ungesättigter Dicarbonsäure verwendet werden.

6. Verfahren nach Ansprüchen 1–5, dadurch gekennzeichnet, daß als ungesättigte Säuren der allg. Formel (I) Fumar- und/oder Maleinsäure verwendet werden.

**Claims**

1. Process for the preparation of (thio)hydantoins consisting of the structural unit II

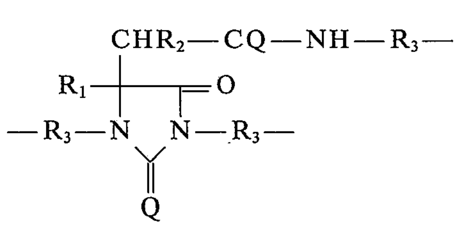

(II)

which optionally recurs 1–1000 times and which is optionally linked through at least one $R_3$ radical and optionally contains masked NCQ end groups, wherein the radicals

$R_1$, $R_2$ and $R_3$, which are identical or different, denote an optionally substituted aliphatic, aliphatic-aromatic, aromatic or heterocyclic radical

| | |
|---|---|
| $R_1$ and $R_2$ | also denote hydrogen and halogen and |
| Q | denotes oxygen or S, |

characterised in that organic iso(thio)cyanates of the general formula (III)

$$R_3(-NCQ)_n$$

or iso(thio)cyanate-releasing compounds are reacted with unsaturated carboxylic acids of the general formula I

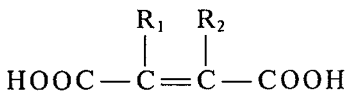

(I)

wherein

| | |
|---|---|
| $R_1$, $R_2$, $R_3$ and Q | have the abovementioned meaning and |
| n | represents an integer from 1 to 4, |

optionally in the presence of a catalyst and one or more solvents at temperatures of −20 to 500° C, at least 2 equivalents of iso(thio)cyanate being used per mol of unsaturated carboxylic acid.

2. Process according to Claim 1, characterised in that

| | |
|---|---|
| $R_1$ and $R_2$, | which are identical or different, denote hydrogen, halogen, an aliphatic radical with $C_1-C_{20}$, an aliphatic-aromatic radical with $C_7-C_{20}$, an aromatic radical with $C_6-C_{20}$ or a heterocyclic radical with 5 to 16 ring members and at least one N, O or S atom in the ring, |
| $R_3$ | denotes an alkyl radical with 2−20 C atoms, an aryl radical with 5−12 C atoms, a cycloalkyl radical with 5−12 C atoms, an alkyl-aryl radical with 6−20 C atoms and an aryl or cycloalkyl radical with 5−12 C atoms containing hetero atoms such as N, O or S, all of which may be substituted by halogen, alkyl with $C_1-C_{10}$ and/or aryl groups with $C_6-C_{12}$. |

3. Process according to Claim 1, characterised in that organic polyiso(thio)cyanates are used.

4. Process according to Claims 1−3, characterised in that organic diiso(thio)cyanates are used.

5. Process according to Claims 1−4, characterised in that 3−6 equivalents of iso(thio)cyanate are used per mol of unsaturated dicarboxylic acid.

6. Process according to Claims 1−5, characterised in that fumaric acid and/or maleic acid are used as the unsaturated acids of the general formula (I).

**Revendications**

1. Procédé de préparation de (thio)hydantoïnes, consistant en le motif de structure II éventuellement répété 1 à 1000 fois

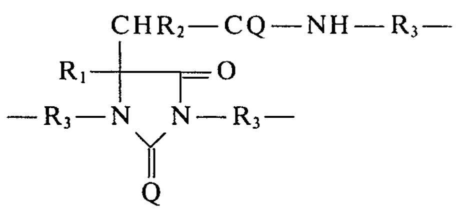

(II)

éventuellement relié par l'intermédiaire d'au moins un reste $R_3$, avec, le cas échéant, des groupes terminaux NCQ bloqués, les restes

| | |
|---|---|
| $R_1$, $R_2$ et $R_3$, | identiques ou différents, représentant un reste aliphatique, aliphatique-aromatique, aromatique ou hétérocyclique éventuellement substitué, |
| $R_1$ et $R_2$ | représentant en outre l'hydrogène et des halogènes et |
| Q | représentant l'oxygène ou le soufre, caractérisé en ce que l'on fait réagir des iso(thio)cyanates organiques de formule générale III |

$$R_3(-NCQ)_n \quad \text{(III)}$$

ou des composés fournissant des iso(thio)cyanates, avec des acides carboxyliques insaturés de formule générale I

$$HOOC-\overset{R_1}{\overset{|}{C}}=\overset{R_2}{\overset{|}{C}}-COOH \qquad (I)$$

| | |
|---|---|
| $R_1$, $R_2$, $R_3$ et Q | ayant les significations indiquées ci-dessus et |
| n | représentant un nombre entier de 1 à 4, |

éventuellement en présence d'un catalyseur et d'un ou plusieurs solvants à des températures allant de $-20$ à 500° C, en utilisant au moins deux équivalents d'iso(thio)cyanate par mole d'acide carboxylique insaturé.

2. Procédé selon la revendication 1, caractérisé en ce que

| | |
|---|---|
| $R_1$ et $R_2$, | identiques ou différents, représentent l'hydrogène, un halogène, un reste aliphatique en $C_1-C_{20}$, un reste aliphatique-aromatique en $C_7-C_{20}$, un reste aromatique en $C_6-C_{20}$ ou un reste hétérocyclique contenant de 5 à 16 chaînons cycliques et au moins un atome de N, O ou S dans le cycle, |
| $R_3$ | représente un reste alkyle en $C_2-C_{20}$, un reste aryle en $C_5-C_{12}$, un reste cycloalkyle en $C_5-C_{12}$, un reste alkylaryle en $C_6-C_{20}$ contenant un hétéroatome tel que N, O ou S, un reste aryle ou cycloalkyle en $C_5-C_{12}$, tous ces restes pouvant être substitués par des halogènes, des groupes alkyle en $C_1-C_{10}$ et/ou aryle en $C_6-C_{12}$. |

3. Procédé selon la revendication 1, caractérisé en ce que l'on utilise des polyiso(thio)cyanates organiques.

4. Procédé selon les revendications 1 à 3, caractérisé en ce que l'on utilise des diiso(thio)cyanates organiques.

5. Procédé selon les revendications 1 à 4, caractérisé en ce que l'on utilise de 3 à 6 équivalents d'iso(thio)cyanate par mole d'acide dicarboxylique insaturé.

6. Procédé selon les revendications 1 à 5, caractérisé en ce que l'on utilise en tant qu'acides insaturés de formule générale I l'acide fumarique et/ou l'acide maléique.